# EUROPEAN PATENT SPECIFICATION

(11) **EP 2 929 892 B1**
(45) Date of publication and mention of the grant of the patent: **04.07.2018**
(21) Application number: 15166651.8
(22) Date of filing: 18.05.2012
(51) Int. Cl.: A61K 38/47, C12N 9/24

(54) **PROCESS FOR PURIFYING HEPARAN-N-SULFATASE**
VERFAHREN ZUR REINIGUNG VON HEPARAN-N-SULFATASE
PROCÉDÉ DE PURIFICATION HÉPARAN-N-SULFATASE

(30) Priority: 19.05.2011 US 201161488090 P
(43) Date of publication of application: 14.10.2015
(62) Divisional of application: 12785579.9
(73) Proprietor: Shire Human Genetic Therapies, Inc., Lexington MA 02421 (US)
(72) Inventor: Nichols, David, Newton, MA 02459 (US)
(74) Representative: Carpmaels & Ransford LLP

(56) References cited:
- US-A1- 2006 234 226
- US-A1- 2009 191 178
- GLIDDON B L ET AL: "Purification and characterization of recombinant murine sulfamidase", MOLECULAR GENETICS AND METABOLISM, ACADEMIC PRESS, AMSTERDAM, NL, vol. 83, no. 3, 1 November 2004 (2004-11-01), pages 239-245, XP004631114, ISSN: 1096-7192, DOI: 10.1016/J.YMGME.2004.07.016
- J Bielicki ET AL: "Recombinant human sulphamidase: expression, amplification, purification and characterization", The Biochemical journal, 1 January 1998 (1998-01-01), pages 145-150, XP055143345, ENGLAND Retrieved from the Internet: URL:http://www.pubmedcentral.nih.gov/artic lerender.fcgi?artid=1219025&tool=pmcentrez &rendertype=abstract [retrieved on 2014-09-29]
- MAHURAN D ET AL: "A rapid four column purification of 2-deoxy-D-glucoside-2-sulphamate sulphohydrolase from human liver", BIOCHIMICA ET BIOPHYSICA ACTA (BBA) - GENERAL SUBJECTS, ELSEVIER, AMSTERDAM, NL, vol. 757, no. 3, 9 June 1983 (1983-06-09), pages 359-365, XP025793331, ISSN: 0304-4165, DOI: 10.1016/0304-4165(83)90062-4 [retrieved on 1983-06-09]
- C Freeman ET AL: "Human liver sulphamate sulphohydrolase. Determinations of native protein and subunit Mr values and influence of substrate agylcone structure on catalytic properties", The Biochemical journal, 15 February 1986 (1986-02-15), pages 83-92, XP055143392, ENGLAND Retrieved from the Internet: URL:http://www.ncbi.nlm.nih.gov/pubmed/370 7548 [retrieved on 2014-09-29]
- Eduard Paschke ET AL: "677 Printed in Great Britain Multiple Forms of 2-Deoxy-D-glucoside-2-sulphamate Sulphohydrolase from Human Placenta", Biochem. J, 1 January 1978 (1978-01-01), pages 677-684, XP055143393, Retrieved from the Internet: URL:http://www.biochemj.org/bj/181/0677/18 10677.pdf [retrieved on 2014-09-29]
- CHEN J ET AL: "The distinctive separation attributes of mixed-mode resins and their application in monoclonal antibody downstream purification process", JOURNAL OF CHROMATOGRAPHY, ELSEVIER SCIENCE PUBLISHERS B.V, NL, vol. 1217, no. 2, 8 January 2010 (2010-01-08), pages 216-224, XP026817104, ISSN: 0021-9673 [retrieved on 2009-09-23]

## Description

### FIELD OF THE INVENTION

The present invention relates to processes and methods for preparing and purifying heparan-N-sulfatase. The disclosed processes and methods generally comprise subjecting heparan-N-sulfatase to one or more chromatographic steps under conditions that yield highly pure heparan-N-sulfatase.

### BACKGROUND

The mucopolysaccharidoses (MPS) are a group of rare, inherited lysosomal storage disorders caused by the deficiency or absence of specific lysosomal enzymes. The absence of these enzymes results in the accumulation of complex sugar molecules in the cells and tissues, as well as in cellular organelles called lysosomes. In the presence of normal lysosomal enzymes these sugars are transformed into other substances and used by the body. These complex sugars are known as mucopolysaccharides or glycosaminoglycans (GAGs) and serve as the building blocks for connective tissues in the body.

MPS III results from the lack of four different enzymes necessary to degrade the GAG. Each enzyme deficiency defines a different form of Sanfilippo syndrome: type IIIA (Sanfilippo A), type IIIB (Sanfilippo B), type IIIC (Sanfilippo C), and type IIID (Sanfilippo D). Heparan-N-sulfatase (HNS) is an enzyme that participates in the stepwise degradation of heparan sulfate. HNS hydrolyzes the sulfate moiety attached to the amino group of the glucosamine residue of heparan sulfate, a type of GAG. A deficiency of this enzyme is associated with mucopolysaccharidoses IIIA (MPS, Sanfilippo's syndrome A). Patients affected by MPS type III A have mutations in the gene coding for HNS, resulting in a deficiency or absence of this enzyme.

Symptoms of MPS IIIA (Sanfilippo A) usually arise between 2 to 6 years of age, although in some cases diagnosis is made as late as 13 years of age. The clinical symptoms of the condition present with differing degrees of severity. The central nervous system is the most severely affected system in patients with MPS IIIA. HNS and other secondarily stored compounds accumulate primarily in the central nervous system. Problems in language development, motor skills, and intellectual development characterize the condition. Overall, individuals with MPS IIIA have a marked developmental delay, and long-term survival is poor. The condition is chronically debilitating and life-threatening.

Presently no approved therapeutic treatments for MPS IIIA are available. Bone marrow transplant has been used in an attempt to slow disease progression. Because heparan sulfate is the natural substrate of HNS, animal studies have shown that HNS may be useful for the treatment of lysosomal storage disorders, such as MPS IIIA, in which there is an increase in heparan sulfate.

Given the interest in HNS as a pharmaceutical agent, there remains a need for preparation of large quantities of highly purified material in a cost effective manner. Various reports of purifying HNS from culture medium have been reported (Hemsley et al., Mol. Genet. Metab. 90:313-328 (2007)). While several methods of purification of HNS have been attempted and described, none have been suitable for production of HNS for use in human therapy of MPS IIIA. For example, Gliddon et al. (Mol Genet Metab. 2004 Nov;83(3):239-45) disclose the purification and characterization of recombinant murine sulfamidase. Bielicki et al. (Biochem J. 1998 Jan 1;329 (Pt 1):145-50) disclose the expression, amplification, purification and characterization of recombinant human sulphamidase. Mahuran et al. (Biochim Biophys Acta. 1983 Jun 9;757(3):359-65) disclose a rapid four column purification of 2-deoxy-D-glucoside-2-sulphamate sulphohydrolase from human liver. Freeman and Hopwood (Biochem J. 1986 Feb 15;234(1):83-92) describe the determinations of native protein and subunit Mr values of human liver sulphamate sulphohydrolase and the influence of substrate agylcone structure on catalytic properties. Paschke and Kresse (Biochem J. 1979 Sep 1;181(3):677-84) describe multiple forms of 2-deoxy-D-glucoside-2-sulphamate sulphohydrolase from human placenta.

### SUMMARY

The invention relates generally to a process for the purification of heparan-N-sulfatase (HNS), in particular the purification of recombinant human HNS (rhHNS) from culture medium or a semi-purified sample of crude recombinant HNS, as well as to compositions and formulations comprising HNS purified by the process and methods of using said purified HNS. Described methods comprise the use of a combination of chromatographic methods to purify HNS. Specifically, the invention relates to a process for the purification of human heparan-N-sulfatase (HNS) that contains reduced amounts of high pI HNS, which comprises (a) contacting a bulk HNS composition containing a mixture of human HNS and other contaminating proteins of a cell culture supernatant medium obtained from a host cell genetically engineered to make human HNS with an anionic exchange chromatography resin; and (b) extracting and/ or purifying HNS from the bulk HNS composition from the anionic exchange chromatography resin by adjusting the pH to 7.0; wherein the process removes from 10% to 25% of high pI HNS present in the bulk HNS composition. A human HNS composition obtained by this process is also provided. In particular, the invention also relates to a human HNS composition that contains reduced amounts of high pI HNS having an HNS concentration above 15 gram per liter, wherein the composition is produced by a process that removes from 10% to 25% of high pI HNS present in bulk HNS compositions. The invention is based on the recognition that the published procedures for isolating HNS do not reproducibly yield HNS of sufficient solubility to be therapeutically useful.

The HNS obtained by the methods of the invention
contains reduced amounts of high pI HNS which may reduce its solubility. The purification process allows for large amount of higher yields of HNS and higher purity of HNS than that provided by known processes. This purification process is particularly useful for preparing pharmaceutical grade HNS for use in humans (e.g., rhHNS).

Methods are provided herein for purifying recombinant human HNS by purifying material extracted from cell culture medium and exposing the extracted material to one or more column chromatography or batch chromatography media (e.g., one, two, three, four, five, six, seven, eight, nine, ten or more). Similarly, methods are provided herein for purifying recombinant human HNS by further purifying an enriched eluate extracted from one or more column chromatography or batch chromatography media by exposing such enriched eluate to one or more additional column chromatography or batch chromatography purification steps (e.g., one, two, three, four, five, six, seven, eight, nine, ten or more).

In one embodiment, HNS is purified using a four column process comprising the purification steps of 1) filtering the initial HNS in solution extracted from the cell culture medium; 2) loading the filtered HNS onto an anion exchange matrix (e.g., Q Sepharose Fast Flow™ column), washing the column and eluting the enriched HNS from the column; 3) loading the eluate from the anion exchange column onto a hydrophobic interaction column (HIC) (e.g., Phenyl Sepharose), washing the column and eluting the enriched HNS from the column; 4) loading the eluate from the HIC column onto a hydroxyapatite column (HA) (e.g., ceramic hydroxyapatite Type 1), washing the column and eluting the enriched HNS from the column; and 5) loading the eluate from the HA column onto a cationic exchange column (e.g., SP Sepharose), washing the column and eluting the enriched HNS from the column. In certain embodiments, the eluate recovered from the cationic exchange column is further filtered and concentrated by ultrafiltration or diafiltration. In certain embodiments, the eluate recovered from the cationic exchange column is further purified (e.g., by loading such eluate into one or more of the anionic exchange column, the HIC column, the HA column and/or the cationic exchange column).

It should be noted that in certain embodiments, the performance of each of the column chromatography purification steps need not necessarily be dependant on the previously performed column chromatography purification step. Accordingly, in certain embodiments the order in which each of the column chromatography purification steps are performed is not critical, and references made in a subsequent column chromatography purification step to, for example, a specific eluate from a previous step, are made for convenience and/or clarity. For example, in certain embodiments, HNS is purified using a four column process, however after filtering the initial HNS in solution extracted from the cell culture medium the recited purification steps are performed in a different order. For example, the purification steps may comprise 1) loading the filtered HNS onto a anion exchange matrix (e.g., Q Sepharose Fast Flow™ column), washing the column and eluting the enriched HNS from the column; 2) loading the eluate from the anion exchange column onto a hydroxyapatite (HA) column (e.g., ceramic hydroxyapatite Type 1), washing the column and eluting the enriched HNS from the column; 3) loading the eluate from the HA column onto a cationic exchange column (e.g., SP Sepharose), washing the column and eluting the enriched HNS from the column; and 4) loading the eluate from the cationic exchange column onto a hydrophobic interaction column (HIC) (e.g., Phenyl Sepharose), washing the column and eluting the enriched HNS from the column).

In certain embodiments, HNS is purified using at least one, at least two, at least three, at least four or more column chromatography purification steps. For example, in certain embodiments, HNS is purified using a three column process wherein after filtering the initial HNS in the solution extracted from the cell culture medium, such filtered HNS is subjected to purification comprising the steps of 1) loading the HNS onto a anion exchange matrix (e.g., Q Sepharose Fast Flow™ column), washing the column and eluting the enriched HNS from the column; 2) loading the eluate from the anion exchange column onto a hydrophobic interaction column (HIC) (e.g., Phenyl Sepharose), washing the column and eluting the enriched HNS from the column; and 3) loading the eluate from the HIC column onto a hydroxyapatite column (HA) (e.g., ceramic hydroxyapatite Type 1), washing the column and eluting the enriched HNS from the column.

While each of the recited column chromatography purification steps may be performed without respect to a previous column chromatography purification step, it should be understood that the individual components which comprise each of the column chromatography purification steps are intended to be performed in the order recited. For example, the step of loading an initial HNS extract (or alternatively an eluate from a previously performed column chromatography purification step) onto a column must precede washing of that column, and washing of the column must precede the elution of the enriched HNS from that column.

In accordance with a further aspect provided herein, a purified recombinant HNS composition is described that can be useful for treating a subject suffering from a lysosomal enzyme deficiency such as MPS IIIA. HNS has been shown to have activity when administered via the cerebrospinal fluid in a naturally occurring mouse model of MPS IIIA. Hemsley, et al., Mol. Genet. Metab. 90:313-328 (2007). Intra-cisternal delivery of HNS in a MPS IIIA Huntaway dog model also showed therapeutic activity. Hemsley, et al., Mol. Genet. Metab. 98(4): 383-92 (2009).

In another aspect, the disclosure provides compositions of HNS that are substantially pure HNS. The purified HNS preparation is greater than about 90% free of contaminants. Preferably, the material is greater than 80%, 85%, 90%, 91%, 92%, 93%, 94%, 95%, 96%, 97%, 98%, or even greater than 99% free of contaminants.

A pharmaceutical composition is disclosed herein that comprises a therapeutically effective amount of purified recombinant HNS as prepared by the process described herein, together with suitable excipients. The pharmaceutical composition of recombinant HNS is particularly suitable for topical, oral or parenteral (e.g., intravenous, subcutaneous, intramuscular or intrathecal) administration to a subject.

The above discussed and many other features and attendant advantages of the present invention will become better understood by reference to the following detailed description of the invention when taken in conjunction with the accompanying examples. The various embodiments described herein are complimentary and can be combined or used together in a manner understood by the skilled person in view of the teachings contained herein.

### BRIEF DESCRIPTION OF THE DRAWINGS

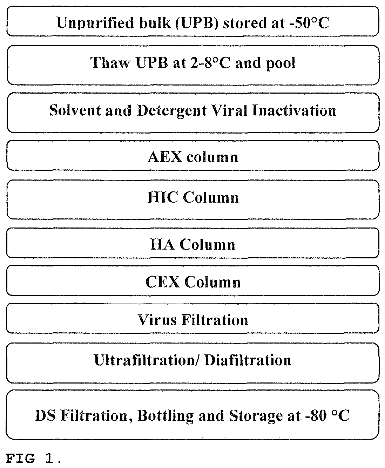

FIG. 1 illustrates a purification process flow diagram of an embodiment provided herein.

### DETAILED DESCRIPTION

Certain embodiments described herein provide methods and processes for preparing purified HNS, a lysosomal enzyme for use in the treatment of MPS IIIA.

Processes for purifying HNS are known in the art. See e.g., Hemsley et al., Mol. Genet. Metab. 90:313-328 (2007); U.S. Patent Application Pub. No. 2009/0186011

However, previously published methods for preparing HNS were not manufacturable, were not easily scaled up and/or do not reproducibly yield pure HNS suitable for use in humans.

The HNS produced by methods described herein is particularly well suited for use as a therapeutic agent (e.g., for the treatment of MPS IIIA).

Heparan-N-sulfatase is a lysosomal enzyme also known in the art by the names N-sulphoglucosamine sulphohydrolase; SGSH; EC 3.10.1.1; N-sulfoglucosamine sulfohydrolase; 2-desoxy-D-glucoside-2-sulphamate sulphohydrolase (sulphamate sulphohydrolase); heparin sulfamidase; sulfoglucosamine sulfamidase; sulphamidase; HNS,rhHNS, sulfamidase, rhNS, and rhSGSH. The term "HNS" as used herein encompasses this enzyme, including functional fragments and/or derivatives thereof, and any pharmaceutically acceptable forms thereof. Heparin-N-sulfatase is associated with Online Mendelian Inheritance in Man (OMIM) identification no. OMIM 605270, the entry for which is publicly available online at http://www.ncbi.nlm.nih.gov/omim/605270.

As used herein, "HNS composition" means any composition containing HNS, in various states of purity.

As used herein, the term "substantially pure" means that the proteins or polypeptides are essentially free of other substances to an extent practical and appropriate for their intended use. In particular, the proteins are sufficiently pure and are sufficiently free from other biological constituents of their hosts cells and viruses so as to be useful in, for example, pharmaceutical preparations. As used herein, a "substantially pure HNS" is a preparation of HNS, which has been isolated or synthesized and which is greater than about 90% free of contaminants. Preferably, the material is greater than 80%, 85%, 90%, 91%, 92%, 93%, 94%, 95%, 96%, 97%, 98%, or even greater than 99% free of contaminants. The degree of purity may be assessed by means known in the art.

The terms "treat" and "treating" as used herein refer to reversing or blocking the progression of the disease in the subject.

Because HNS is a naturally occurring enzyme, it is typically prepared by isolation from a cell culture supernatant medium obtained from a host cell suitable for making the protein. In certain embodiments the host cell is genetically engineered to produce HNS. For example, the genes responsible for the cellular machinery that produce HNS can be placed into a microorganism such as bacteria or fungi. In other embodiments, the genes responsible for the cellular machinery that produce HNS can be placed into a mammalian cell. Non-limiting examples of mammalian cells that may be used include BALB/c mouse myeloma line (NSO/l, ECACC No: 85110503); human retinoblasts (PER.C6, CruCell, Leiden, The Netherlands); monkey kidney CV1 line transformed by SV40 (COS-7, ATCC CRL 1651); human embryonic kidney line (293 or 293 cells subcloned for growth in suspension culture, Graham et al., J. Gen Virol., 36:59,1977); human fibrosarcoma cell line (HT1080); baby hamster kidney cells (BHK, ATCC CCL 10); Chinese hamster ovary cells +/-DHFR (CHO, Urlaub and Chasin, Proc. Natl. Acad. Sci. USA, 77:4216, 1980); mouse sertoli cells (TM4, Mather, Biol. Reprod., 23:243-251, 1980); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68, 1982); MRC 5 cells; FS4 cells; and a human hepatoma line (Hep G2) .

In certain aspects provided herein, the culture conditions for the host cells are optimized to produce a high level of HNS with minimal levels of contaminants. In another aspect provided herein, the process of purifying HNS is intended for use with biological materials, particularly crude mixtures containing HNS and other contaminating proteins, referred to as starting material samples or bulk material. In accordance with one aspect provided herein, a method for the purification of HNS is described, in particular for the purification of recombinant human HNS (rhHNS), from a crude preparation of the culture medium of the recombinant process or bulk material. The rhHNS obtained by this method has a high degree of purity and high specific bioactivity (e.g., in the range of at least 10 units/mg, at least 15 units/mg, at least 20 units/mg, at least 25 units/mg, at least 30 units/mg, at least 35 units/mg, at least 40 units/mg, at least 45 units/mg, at least 47 units/mg, at least 50 units/mg, at least 60 units/mg, at least 70 units/mg, at least 75 units/mg, at least 85 units/mg, at least 90 units/mg, at least 100 units/mg, or more), and is practically free from host cell proteins which are present in the culture medium and from nucleic acids or other contaminants contained in the host cells used in the recombinant process.

In one embodiment, the sample of HNS is initially constituted by collecting cell culture supernatant medium. It is contemplated that the crude solution may be filtered or concentrated and subjected to one or more steps to remove contaminants derived from the cell culture to yield bulk material. The purification process as described herein may include one or more subsequent chromatography steps (e.g., at least one, at least two, at least three, at least four, at least five, at least six, at least seven, at least eight, at least nine, at least ten, or more chromatography steps) in order to achieve a desired degree of purity of HNS.

In certain embodiments the semi-purified material is first captured by exposure to mercapto-ethyl-pyridine. In one embodiment the mercapto-ethyl-pyridine is 4-mercapto-ethyl-pyridine linked to a cellulose matrix.

In another embodiment, the HNS material is subjected to viral inactivation prior to being further purified. Viral inactivation may be accomplished, for example by adding 1% Tween 80 and 0.3% TnBP to in-process HNS samples or media and holding at ambient temperature for 3-16 hours. This step may be performed at any point in the purification scheme. Further, filtration of the HNS composition using a 0.2 µm filter may be incorporated into any loading step.

In yet other embodiments, the resulting HNS material is optionally reduced in volume prior to column chromatography purification. In other embodiments, the volume is reduced following recovery of the enriched HNS eluate following the column chromatography steps.

In certain embodiments, methods and processes for purifying HNS by a sequence of chromatography steps are included.

To the extent the multiple column chromatography steps are disclosed, such steps need not be performed sequentially or in the recited order. For example, in certain embodiments, the HNS is purified using at least one, at least two, at least three, at least four or more column chromatography purification steps. Similarly, in certain embodiments one or more of the recited column chromatography steps may be performed multiple times. In some embodiments, one or more of the chromatography steps includes loading, equilibrating, washing, and eluting of the chromatography medium or resin. Notwithstanding the foregoing statements regarding the sequential performance of each of the chromatography purification steps, it should be understood that the individual components which comprise each of the column chromatography purification steps are intended be performed in the order recited. For example, as will be appreciated by one of skill in the art, the steps of loading, equilibrating, washing, and eluting which generally comprise each chromatography purification step are intended to be performed in the recited order.

Typically, a HNS composition is contacted with a series of chromatographic media during purification. In accordance with
the invention, the chromatography media or resins include one or more anionic exchange resin.

The chromatography media or resin may also include one or more hydrophobic interaction resin.

The chromatography media or resin may also include one or more hydroxyapatite resin.

The chromatography media or resin may also include one or more cationic exchange resin.

In certain embodiments, the chromatography media or resins include an anionic exchange resin, a hydrophobic interaction resin, a hydroxyapatite resin, and a cationic exchange resin. In certain embodiments, the extracted material is purified using a column packed with Q Sepharose, followed by a column packed with Phenyl Sepharose, followed by a column packed with ceramic hydroxyapatite Type I; and finally followed by another column packed with SP Sepharose. The contemplated steps for purifying the extracted material need not all be performed. For example, in certain embodiments the extracted material is purified using a column packed with Q Sepharose, followed by a column packed with Phenyl Sepharose, followed by a column packed with ceramic hydroxyapatite Type I. Similarly, the contemplated steps for purifying the extracted material need not all be performed in any particular order. For example, in certain embodiments, the extracted material is purified using a column packed with Q Sepharose, followed by a column packed with Phenyl Sepharose, followed by another column packed with SP Sepharose; finally followed by a column packed with ceramic hydroxyapatite Type I. In each of the forgoing embodiments, each of the columns is optionally washed with buffered or other aqueous solution followed by elution of HNS using an aqueous solution. In certain embodiments, the HNS composition is eluted from the chromatography medium between each step. It is contemplated that each elution step may be repeated one or more times before advancing to the next purification step. In certain embodiments the extracted material is further purified by filtration. In yet other embodiments, the extracted material is subjected to viral inactivation before, after or during chromatography. In accordance with the invention, the chromatography media or resins comprise an anionic exchange resin. In certain embodiments, contacting the HNS composition with the anionic exchange chromatography resin is, for example, the first, second, third or fourth chromatographic step. Various chromatographic resin or medium may be employed, including, for example, resins from GE HealthCare, Tosoh Biosciences, Applied Biosystems, Bio-Rad, and Pall. Examples of suitable anionic exchange chromatography media are diethylaminoethyl (DEAE), quaternary aminoethyl (QAE) or quaternary ammonium (Q) resin. In certain embodiments, the anionic exchange chromatography resin is a Q sepharose fast flow resin.

The process of purification of HNS may also comprise ahydrophobic interaction chromatography (HIC) step. In some embodiments, the HNS composition is contacted with a hydrophobic interaction chromatography resin as an intermediate step in the purification process. In other embodiments, contacting the HNS composition with the hydrophobic interaction chromatography resin is, for example, the first, second, third or fourth chromatographic step. Examples of suitable hydrophobic interaction chromatography media include phenyl, octyl, butyl, hexyl, propyl, PPG, or ether. In certain embodiments, purification of the HNS extract is performed using a Phenyl Sepharose 6 Fast Flow column. In certain embodiments, the HNS composition or eluate resulting from contact with the hydrophobic interaction chromatography resin is further contacted with a hydroxyapatite chromatography resin.

The chromatography media or resin may also comprise a hydroxyapatite (HA) resin. In some embodiments, contacting the HNS composition with the hydroxyapatite resin is, for example, the first, second, third or fourth chromatographic step. In some embodiments, the extract containing HNS is purified using a column packed with ceramic hydroxyapatite Type I. In some embodiments, the extract containing HNS is purified using a column packed with ceramic hydroxyapatite Type II. In yet another embodiment the HNS composition or eluate collected from the interaction with the hydroxyapatite chromatography resin is further contacted with a cationic exchange chromatography resin.

In certain embodiments, the HNS composition is further purified using a cationic exchange chromatography step. In certain embodiments, the purification using a cationic exchange chromatography step is an intermediate step in the purification of HNS. In other embodiments, contacting the HNS composition with the cationic exchange chromatography resin is the first, second, third, forth or last chromatographic step. In some embodiments, the chromatography media or resin comprises a cationic exchange resin. Examples of suitable cationic exchange chromatography media include chromatography media such as carboxymethyl (CM), sulfopropyl (SP) or methyl sulfonate (S). In some embodiments, the cationic exchange chromatography resin is a SP sepharose fast flow resin.

In one embodiment the HNS obtained following the cationic exchange step is further filtered. In certain embodiments, the HNS is further filtered by, for example, diafiltration or ultrafiltration.

In one step, the purification occurs when the material containing the crude HNS is loaded onto a matrix and pre-equilibrated. The matrix is then washed to remove impurities. It is contemplated that column characteristics may be altered in bore size and length to allow elution with various gradients. As will be appreciated by one of skill in this art, the washing and elution solvents are determined by the matrix used and the polarity of the HNS in such an environment.

Extraction and/or purification of HNS from the bulk HNS composition from an anionic exchange chromatography resin can be optimized upon adjustment of pH levels.

A pH level of 7.0 has been shown to optimize extraction and purification. In accordance with the
invention, the pH of the unpurified bulk HNS composition is adjusted to a pH of 7.0 prior to contacting the HNS with the anionic exchange chromatography resin. In certain embodiments, the material to be loaded on the anion exchange column is adjusted from about 50 mM to about 100 mM NaAcetate. In some embodiments, the solution containing the HNS composition to be loaded on the anionic exchange resin has a sodium acetate concentration from about 50 to about 100 mM. It has been determined that a conductivity of from about 3-4 mS/cm of the HNS composition facilitates the removal of high pI HNS species using anionic exchange chromatograph resins. Accordingly, in certain embodiments, the conductivity of the HNS composition is adjusted to obtain a conductivity of from about 3 to about 4 mS/cm prior to contacting the HNS composition with anionic exchange chromatography resin. In another embodiment, the conductivity is adjusted to about 3.5 mS/cm prior to contacting the HNS composition with the anionic exchange chromatography resin. In certain embodiments, the HNS composition is viral inactivated prior to loading on the anionic exchange column. In yet another embodiment, the HNS composition is filtered using a 0.2 µm filter prior to loading on the anionic exchange column.

In one embodiment, the anionic exchange column is washed with about 5 column volumes of a buffer containing about 20 mM MES-Tris and about 20 mM NaCl at a pH of about 7.0 prior to elution of the enriched HNS composition from the anion exchange column. In certain embodiments there are additional elution steps between contacts with each chromatography resin. In one embodiment, the HNS is eluted from the anionic exchange chromatography resin using a buffer constituting about 20mM MES-Tris and about 180mM NaCl at about pH 7.0. In certain embodiments, the percent recovery of the enriched HNS in the flow through and wash is measured by absorbance units, enzyme activity or ELISA. In one embodiment, the host cell protein clearance is about two fold after this step. The process removes from 10 to 25% of a high pI HNS.

The removal of the high pI HNS leads to improved solubility.

In certain embodiments, the hydrophobic interaction resin is equilibrated with a buffer comprising about 20 mM MES-Tris and a NaCl concentration of about 1.1 to 1.5 M, at a pH of about 7.0 and a conductivity of from about 90 to about 120 mS/cm prior to contacting the HNS composition with the hydrophobic interaction column. Such concentrations, pH and conductivity facilitate the binding of HNS to the hydrophobic interaction column, thereby optimizing the purification of the HNS composition.

In certain embodiments, the eluate from the anionic exchange chromatography step containing enriched HNS is the starting material for the hydrophobic interaction step. In one embodiment, the NaCl concentration of the HNS composition is adjusted to achieve a NaCl concentration of from about 1.1 M to about 1.5 M NaCl prior to contacting the HNS composition with the hydrophobic interaction column. In another embodiment, the NaCl concentration is adjusted to about 1.2 M prior to contacting the HNS composition with the hydrophobic interaction column. The pH of the HNS composition is adjusted to about 7.0 prior to being contacted with the hydrophobic interaction column. In some embodiments, the HNS composition is adjusted to obtain a conductivity of from about 85 to 120 mS/cm at 25°C prior to contacting the HNS composition with the hydrophobic interaction column. In some embodiments, the HNS composition is adjusted to obtain a conductivity of from about 90 to 110 mS/cm at 25°C prior to contacting the HNS composition with the hydrophobic interaction column.

In certain embodiments, the HNS composition adsorbed to the hydrophobic interaction resin is washed with 4 column volumes of a buffer comprising about 20 mM MES-Tris to wash out impurities and a NaCl concentration of from about 1.1M to about 1.5M, at a pH of about 7.0. In yet another embodiment, the NaCl concentration is about 1.2M.

In one embodiment, the hydrophobic interaction column is eluted with about 4 column volumes of a buffer containing about 20 mM MES-Tris and about 180 to 220 mM NaCl at a pH of about 7.0 to elute the enriched HNS composition from the hydrophobic interaction column. In certain embodiments there are additional elution steps. In one embodiment, the HNS is eluted from the hydrophobic interaction chromatography resin using a buffer constituting about 20mM MES-Tris and about 200 mM NaCl at about pH 7.0 with a conductivity range from about 19 to about 23 mS/cm at 25°C to optimize the recovery of purified HNS. In another embodiment, the pH range is from about 6.9 to 7.1. In certain embodiments, the percent recovery of the enriched HNS in the flow through and wash is measured by absorbance units, enzyme activity or ELISA. In one embodiment, the host cell protein clearance is about 35 to 45 fold after this step.

In certain embodiments, pooled eluates of enriched HNS obtained from the hydrophobic interaction column may be used as the starting material for purification employing a hydroxyapatite column. In some embodiments, the solution containing the HNS composition after elution from the hydrophobic interaction column is adjusted to a concentration of about 2 mM to about 4 mM of NaPO₄ to optimize purification. In certain embodiments, the concentration of NaPO4 is adjusted to about 2 mM and a pH of about 7.0 + 0.1. In one embodiment, the equilibration buffer contains about 20mM MES-Tris and about 200 mM NaCl at about pH 7.0. In certain embodiments, the pH of the equilibration buffer is adjusted to from about 7.0 to about 7.2. In yet another embodiment, the HNS composition is filtered using a 0.2 µm filter prior to loading on the anionic exchange column. In another embodiment, the equilibration buffer contains about 2mM NaPO₄, about 20 mM MES-Tris and about 200 mM NaCl at a pH of about 7.0.

In one embodiment, the hydroxyapatite column is washed with about 4 column volumes of a buffer containing about 2mM to about 4 mM of NaPO4, about 20 mM MES-Tris and about 200 mM NaCl at a pH of from about 7.0 to about 7.2 prior to elution of the enriched HNS composition from the hydroxyapatite column. In another embodiment, the wash buffer contains about 2 mM NaPO₄, about 20 mM MES-Tris and about 200 mM NaCl at a pH of about 7.0.

In some embodiments, the HNS contacted with the hydroxyapatite column is eluted with a solution containing about 25 mM NaPO₄ at a pH of about 7.4 to about 7.6. In another embodiment, the HNS loaded onto the hydroxyapatite column is eluted with an eluent containing from about 20 mM NaPO₄ to about 30 mM NaPO₄ at a pH of about 7.0 to about 7.6. In one embodiment, the elution buffer contains about 20 mM NaPO₄, about 25 mM MES-Tris at a pH of about 7.5 + 0.1. In certain embodiments, the elution step may be repeated at least once. In certain embodiments, the percent recovery of the enriched HNS in the flow through and wash is measured by absorbance units, enzyme activity or ELISA.

In certain embodiments, pooled eluates of enriched HNS obtained from the hydroxyapatite column may be used as the starting material for purification employing a cationic exchange column. In yet another embodiment, the HNS composition in the starting material is adjusted to obtain a conductivity of about 3 to about 4 mS/cm prior to loading on the cationic exchange column to optimize binding of HNS to the cationic resin. In some embodiments, the conductivity is adjusted to about 3 mS/cm and the solution comprises about 20mM sodium acetate at about pH 5.0 to optimize binding of HNS to the cationic column. In yet another embodiment, the conductivity of the HNS composition loaded on the cationic exchange resin is about 4 mS/cm and the solution contains about 40 mM sodium acetate at about pH 5.0 to optimize binding of HNS to the cationic column. In another embodiment, the conductivity of the HNS composition loaded on the cationic exchange resin is about 3.5 mS/cm + 0.5 and the pH is about 5.0. In another embodiment, the HNS composition is filtered using a 0.2 µm filter prior to loading on the cationic exchange column.

In one embodiment, the equilibration buffer contains about 50 mM NaAcetate, from about 20 to about 40 mM NaCl and a pH of about 5.0. In certain embodiments, the pH of the equilibration buffer is adjusted to from about 4.9 to about 5.1. In another embodiment, the equilibration buffer contains about 50 mM NaAcetate, about 20 mM NaCl, a pH of about 5.0, and a conductivity range from about 5 to about 7 mS/cm.

In one embodiment, the cationic exchange column is washed with about 4 column volumes of a buffer containing about 50 mM NaAcetate, from about 20 mM to 40 mM NaCl at a pH of from about 5.0 to about 7.2 prior to elution of the enriched HNS composition from the cationic exchange column. In another embodiment, the wash buffer contains about 50 mM NaAcetate, about 20 mM NaCl, a pH of about 5.0, and a conductivity range from about 5 to about 7 mS/cm.

In some embodiments, the elution of the HNS from the cationic exchange resin is carried out with an eluent comprising about 50 mM sodium acetate and from about 90 mM to about 100 mM NaCl at a pH of about 4.9 to about 5.1. In certain embodiments, the elution of the HNS from the cationic exchange resin is carried out with an eluent comprising about 50 mM sodium acetate and about 90 mM NaCl, at a pH of about 5.0 + 0.1. In certain embodiments, the eluent has a conductivity range of from about 12 to about 14 mS/cm. In certain embodiments, the elution step may be repeated at least once. In certain embodiments, the percent recovery of the enriched HNS in the flow through and wash is measured by absorbance units, enzyme activity or ELISA.

Another embodiment described herein is a purified HNS which has been isolated by the methods above to a level of purity that is greater than about 90% free of contaminants. Preferably, the material is greater than 80%, 85%, 90%, 91%, 92%, 93%, 94%, 95%, 96%, 97%, 98%, or even greater than 99% free of contaminants. The degree of purity may be assessed by any suitable means known in the art.

Products described herein can be useful for treating and/or preventing any disease/condition in a subject whereby glycosaminoglycans have been found to be important in the development and/or progression of the disease. Certain embodiments can be particularly useful for treating and/or preventing any disease or condition in a subject whereby HNS is either non-functional or absent. Treating a disease also includes exacting a desired improvement in the disease or symptoms of the disease.

The compositions disclosed herein may be used alone or in combination with another therapeutic agent for treating a disease associated with mucopolysaccharoidosis or its sequellae in a subject. These additional therapeutic agents can be administered prior to administration of the composition, or they can be administered at the same time or after administration of the composition. Subjects can be, for example, any human or non-human vertebrate, e.g., dog, cat, horse, cow, pig.

The formulation buffers for the purified HNS compositions can be a phosphate buffer, such as 5mM Sodium Phosphate, 145mM NaCl, pH 7.0. Other suitable buffers are known to the skilled artisan.

The final HNS concentration in HNS compositions of the invention is
above 15 gram per liter, e.g. above 20 grams per liter.

Purified HNS compositions described herein may be administered topically (including ophthalmic and to mucous membranes including vaginal and rectal delivery), pulmonary (e.g., by inhalation or insufflation of powders or aerosols, including by nebulizer; intratracheally, intranasally), orally or parenterally. In certain embodiments parenteral administration is preferred and includes intravenous, intraarterial, subcutaneous, intraperitoneal intramuscular, intracranial, intrathecal or intraventricular, administration.

The embodiments described herein will be further illustrated by the following Examples, which should not be construed as limiting. The articles "a" and "an" as used herein in the specification and in the claims, unless clearly indicated to the contrary, should be understood to include the plural referents. Claims or descriptions that include "or" between one or more members of a group are considered satisfied if one, more than one, or all of the group members are present in, employed in, or otherwise relevant to a given product or process.

Where elements are presented as lists, (e.g., in Markush group or similar format) it is to be understood that each subgroup of the elements is also disclosed, and any element(s) can be removed from the group. It should be understood that, in general, where embodiments are
referred to as comprising particular elements, features, etc., certain embodiments
consist, or consist essentially of, such elements, features, etc. For purposes of simplicity those embodiments have not in every case been specifically set forth in so many words herein.

### Exemplification

### Example I

### Purification of Human HNS

The objective of the present studies was to obtain a large quantity of recombinant human HNS (with increased solubility). Stably transfected HT1080 cells were grown under bioreactor culture conditions, and active HNS enzyme was purified from the cell medium. The liquid chromatography apparatus used were the AKTA Explorer Chromatography System from GE Healthcare ((Piscataway, NJ), Model: 18-1403-00) and the Genesys 6 UV-Vis Spectrophotometer from Thermo Scientific ((Waltham, MA), Catalog #335908000, Serial 2M6F078007). The following chromatography resins were employed: Q Sepharose Fast Flow from GE Healthcare ((Piscataway, NJ), catalog #17-0510-04); Phenyl Sepharose 6 Fast Flow from GE Healthcare ((Piscataway, NJ), catalog #17-0973-04); SP Sepharose Fast Flow from GE Healthcare ((Piscataway, NJ), catalog #17-0729-04); and Ceramic Hydroxyapaptite, Type I, 80 µm particle size, from Bio-Rad ((Hercules, CA), catalog #157-0085).

The chromatography columns used in this example include Kontes 30 X 1.0 cm Column from Kontes Glas ((Vineland, NJ), Catalog #420830-3000); XK 16/40 Column from GE Healthcare ((Piscataway, NJ), catalog #18-8774-01); XK 5/30 Column from GE Healthcare ((Piscataway, NJ), catalog #18-8751-01)); and Omnifit 10 x 25 mm Column from Bio-Chem Valve ((Boonton, NJ), Catalog # 006CC-10-02-AF).

A sandwich-based ELISA assay utilizing goat antibodies custom-generated by Cygnus Technologies against the HT1080 host cell lysates was used to determine the host cell protein concentration. Fifty microliters of samples diluted in sample diluent (20 mM sodium phosphate, 0.1% ProClin 300, pH 6.0), assay control (75 ng/ml) and standards are simultaneously incubated with 100 µl of HRP-conjugated antibody (1:95 dilution in conjugate diluent: Cygnus Technologies HRP Conjugate Diluent with 3 mg/ml normal goat IgG) on a precoated micro-ELISA plate for 2 hours at ambient temperature on a Titer Plate shaker.

Contents of the wells were removed at the end of incubation and the plate washed 4 times with ELISA wash buffer (25 mM Tris-HCl with 0.425% NaCl (w/v), 0.025% ProClin 300, pH 7.2). One hundred microliters of TMB substrate solution (tetramethyl benzediene; BioFx TMBW1000-01) was then added to each well and the plate incubated for another 30 minutes. The colorimetric reaction was terminated by the addition of 100 µl of stop solution (0.5 N H₂SO₄) and the absorbance at 450 nm measured using SPECTRAmax PLUS 384 Microplate Spectrophotometer with background subtraction set at 650 nm. A standard curve (0-200 ng/ml) was constructed using SoftMax 4.8 software and HCP concentration in the samples intrapolated. Using this assay, the amount of HCP originating from the HT1080 cell line in HNS samples was quantified.

TK1315 rabbit polyclonal antibody specific for HNS in-house IgG purified was coated at 5.0 ug/mL on a MaxiSorp Nunc Immuno plate for one hour at 37°C. After washing the plate three times with phosphate-buffered saline with 0.05% Tween 20 (PBST), the wells were blocked with 2% bovine serum albumin (BSA) in PBST. Samples and reference standards at appropriate dilution were incubated for one hour at 37°C. After washing the plate four times with PBST, the secondary antibody, TK1315 rabbit polyclonal antibody specific for HNS in-house IgG purified-HRP conjugate (1:3000) was applied. After incubation at 37°C for 30 minutes, the plate was washed three times with PBST. TMB substrate (Bio-Rad, Hercules, CA) was applied, and the plate was incubated for 15 minutes at 37°C before stopping the reaction with 2 M sulphuric acid. The plate was read at 450 nm, and a quadratic curve fit was used to generate the standard curve. This assay was used to quantify the amount of HNS in the samples. The concentration of the purified HNS protein was measured by A₂₈₀ absorbance using a Genesys 6 UV-Vis Spectrophotometer.

A two step HNS activity assay utilizing 4-methylumbelliferyl 2-sulfamino-2-deoxy-alpha-D-glucopyranoside as substrate was used to determine HNS activity. Ten microliters of samples diluted in substrate/reaction buffer was added to the assay plate (Costar 96 well plate, Corning #3912), followed by 20 µl of substrate solution (20 mM 4-methylumbelliferyl 2-sulfamino-2-deoxy-alpha-D-glucopyranoside in substrate/reaction buffer). The plate was incubated at 37°C for one hour in Jitterbug (Boekel Scientific) with mixer setting at 1 for the initial 3 minutes. At the end of incubation, 6 µl of Pi/Ci stop buffer was added and the plate mixed for 3 minutes in Jitterbug with mixer setting at 1 to stop the first step of the reaction. Forty-six microliters of standards (0-50 uM 4-methylumbelliferone in substrate/reaction buffer; 0-2300 picomole) was then added to the first 2 blank rows of the plate followed by the addition of ten microliters of a-glucodidase (250U per ml in 0.2% deactivated BSA) to the wells that contain samples and substrate/reaction buffer blanks (not standards). The plate was incubated at 37°C for another hour in Jitterbug with mixer setting at 1 for the initial 3 minutes. At the end of incubation, 200 µl of Carbonate stop buffer (0.5 M sodium carbonate, pH 10.7, 0.025% Triton-100) was added to the wells that contain standards, samples and blanks to terminate the reaction.

The content of each well was mixed three times with the "shake plate" function using SpectraMax M2 multi-detection microplate reader and the fluorescence at 460 nm measured. A standard curve (0-2300 picomole) was constructed using Microsoft Excel and HNS activity in the samples intrapolated. One activity unit is defined as producing 1 picomole of 4-methylumbelliferone in one hour at 37°C. HNS activity was calculated and expressed as U/ml (or U/mg if the protein concentration is known). Using this assay, the activity values of HNS samples were quantified.

The process for purification consisted of using BioSepra™ MEP HyperCel sorbent (Pall Life Sciences, P/N # 12035-036) capture to yield unpurified bulk material.

Prior to performing the Q column step, a viral inactivation process was performed by adding 1% Tween 80 and 0.3% TnBP to the unpurified bulk and holding the mixture at ambient for 3 to 16 hours. After viral inactivation, the mixture was filtered with a 0.2 um filter. Q load conductivity at 3.0, 3.5 and 4.0 mS/cm was studied. The operational conditions for the three runs are summarized in Table 1. The bulk load material for the Q runs was adjusted to 100mM NaAcetate. The flow rate for the Q runs was 150 cm/hour.

**TABLE 1 Operation Conditions for the Q Runs - Load Evaluation**

| Process Steps | Q load at 3.5 mS/cm | Q load at 3.0 mS/cm (low conductivity) | Q load at 4.0 mS/cm (high conductivity) |
|---|---|---|---|
| Column Size* Unpurified bulk (UPB) Sanitization Equilibration Load | 5.0 X 17.7 cm | 2.6 X 16.5 cm MEP Capture Eluates 0.5 M NaOH | 2.6 X 16.5 cm |
| | 20 mM MES-Tris, 20 mM NaCl, pH 7.0 | | |
| | • Adjusted to 100 mM NaAcetate and pH 7.0 | • Adjusted to 100 mM NaAcetate and pH 7.0 | • Adjusted to 100 mM NaAcetate and pH 7.0 |
| | • viral inactivated | • viral inactivated | • viral inactivated |
| | • adjusted conductivity to 3.5 mS/cm | • adjusted conductivity to 3.0 mS/cm | • adjusted conductivity to 4.0 mS/cm |
| | • 0.2 µm filtered | • 0.2 µm filtered | • 0.2 µm filtered |
| | • loaded at about 3 mg HNS/mL resin | • loaded at about 3 mg HNS/mL resin | • loaded at about 3 mg HNS/mL resin |
| Wash | 5 CV of 20 mM MES-Tris, 20 mM NaCl, pH 7.0 | | |
| Elute | • 20 mM MES-Tris, 180 mM NaCl, pH 7.0 | | |
| | • collect based on 100 mAU - 50 mAU setpoints | | |
| Strip | 3 CV of 20 mM MES-Tris, 1M NaCl | | |
| Clean | 3 CV of 1M NaOH, 2 M NaCl | | |

| | | | |
|---|---|---|---|
| *The results from the 5 cm columns should be comparable to that from 2.6 cm column. The flow rate for the Q runs is 150 cm/hour. | | | |

The results from the Q runs are summarized in Table 2

**TABLE 2 Results of the Q runs - Load evaluation**

| | Loading Condition | | FT/Wash + PrePeak (<100 mAU) | | | | | |
|---|---|---|---|---|---|---|---|---|
| | | | % Recovery by AU | | % Recovery by Activity | | % Recovery by ELISA | |
| Q (low conductivity) | 3.0 mS/cm | | 56.8 | | 13.0 | | 17.8 | |
| Q (control) | 3.5 mS/cm | | 54.9 | | 13.4 | | 20.1 | |
| Q (high conductivity) | 4.0 mS/cm | | 49.1 | | 13.9 | | Sample not tested | |
| | | | | | | | | |

| | Eluate (100 mAU - 50 mAU) | | | | | | | |
|---|---|---|---|---|---|---|---|---|
| | Volume (CV) | % Recovery by AU | | % Recovery by Activity | | % Recovery by ELISA | | HCP Fold Clearance |
| Q (low conductivity) | 1.5 | 35.1 | | 83.6 | | 75.6 | | 2 |
| Q (control) | 1.6 | 40.4 | | 70.0 | | 79.9 | | 2 |
| Q (high conductivity) | 1.2 | 37.2 | | 71.1 | | 64.5 | | 2 |

The Q process was designed to remove 10-25% of high pI HNS to improve the solubility of HNS drug substance. Based on the results from the Q evaluation runs (Table 2), loading the Q column at 3.0, 3.5, and 4.0 mS/cm resulted in similar HNS loss in Q FT/Wash and similar HCP clearance of the eluate. There was some variation in eluate recovery by activity and ELISA, which may be caused by assay variations. It was expected that the recoveries would be similar for all three runs since there was similar HNS loss in the FT/wash. All recoveries by AU, activity, and ELISA were considered acceptable. Based on these results, the conductivity of the loading material was set to 3.5 ± 0.5 mS/cm.

### Example 2

### Phenyl Sepharose Column

Phenyl loading at 1.1 M, 1.2 M, 1.3M, and 1.5M NaCl was studied. Phenyl elution at 180mM, 200mM, and 220 mM NaCl was also studied. The pH of the Phenyl load was not tested, as the HIC column is not expected to be sensitive to the pH changes in the loading process (pH 7.0 ± 0.1). The operational conditions for the Phenyl runs are summarized in Tables 3 and 4. The flow rate for the Phenyl runs was 150 cm/hour.

**TABLE 3 Operation Conditions of the Phenyl Runs - Load Evaluation**

| Process Steps | Phenyl Load at 1.1 M NaCl | Phenyl Load at 1.2 M NaCl (Control) | Phenyl Load at 1.3 M NaCl | Phenyl Load at 1.5 M NaCl |
|---|---|---|---|---|
| Column Size | 1.6 X 17.7 cm | | | |
| Starting Material | Q Eluate | | | |
| Sanitization | 0.5 M NaOH | | | |
| Equilibration | 20 mM MES-Tris, 1.1 M NaCl, pH 7.0 | 20 mM MES-Tris, 1.2 M NaCl, pH 7.0 | 20 mM MES-Tris, 1.3 M NaCl, pH 7.0 | 20 mM MES-Tris, 1.5 M NaCl, pH 7.0 |
| Load | • Adjusted Q eluate to 1.1 M NaCl and pH 7.0 | • Adjusted Q eluate to 1.2 M NaCl and pH 7.0 | • Adjusted Q eluate to 1.3 M NaCl and pH 7.0 | • Adjusted Q eluate to 1.5 M NaCl and pH 7.0 |
| | • 0.2 µm filtered | • 0.2 µm filtered | • 0.2 µm filtered | • 0.2 µm filtered |
| | • loaded at 5.7 AU/mL resin | • loaded at 5.7 AU/mL resin | • loaded at 5.7 AU/mL resin | • loaded at 5.7 AU/mL resin |
| Wash | 4 CV of 20 mM MES-Tris, 1.1 M NaCl, pH 7.0 | 4 CV of 20 mM MES-Tris, 1.2 M NaCl, pH 7.0 | 4 CV of 20 mM MES-Tris, 1.3 M NaCl, pH 7.0 | 4 CV of 20 mM MES-Tris, 1.5 M NaCl, pH 7.0 |
| Elute | • 20 mM MES-Tris, 200 mM NaCl, pH 7.0 | | | |
| | • collect based on 100 mAU - 50 mAU setpoints | | | |
| Strip 1 | 3 CV of water 2 CV of 20% ethanol 2 CV of 0.5 M NaOH | | | |
| Strip 2 | | | | |
| Clean | | | | |

| | | | | |
|---|---|---|---|---|
| The flow rate for the Phenyl runs is at 150 cm/hour. | | | | |

**TABLE 4 Operation Conditions of the Phenyl Runs Elution Evaluation**

| Process Steps | Phenyl elution at 180 mM NaCl | Phenyl elution at 200 mM NaCl (Control) | Phenyl elution at 220 mM NaCl |
|---|---|---|---|
| Column Size | 1.6 X 17.7 cm | | |
| Starting Material | Q Eluate | | |
| Sanitization | 0.5 M NaOH | | |
| Equilibration | 20 mM MES-Tris, 1.2 M NaCl, pH 7.0 | | |
| Load | • Adjusted Q Eluate to 1.2 M NaCl and pH 7.0 | | |
| | • 0.2 µm filtered | | |
| | • Loaded at 5.7 AU/mL resin | | |
| Wash | 4 CV of 20 mM MES-Tris, 1.2 M NaCl, pH 7.0 | | |
| Elute | • 20 mM MES-Tris, 180 mM NaCl, pH 7.0 | • 20 mM MES-Tris, 200 mM NaCl, pH 7.0 | • 20 mM MES-Tris, 220 mM NaCl, pH 7.0 |
| | • Collect based on 100 mAU - 50 mAU setpoints | • Collect based on 100 mAU - 50 mAU setpoints | • Collect based on 100 mAU - 50 mAU setpoints |
| Strip 1 | 3 CV of water | | |
| Strip 2 | 2 CV of 20% ethanol | | |
| Clean | 2 CV of 0.5 M NaOH | | |

| | | | |
|---|---|---|---|
| The flow rate for the Phenyl runs is at 150 cm/hour. | | | |

The results from the Phenyl runs are summarized in Tables 5 and 6.

**TABLE 5 Results of the Phenyl Runs - Load Evaluation**

| | Conductivity (mS/cm) | FT/Wash + PrePeak (< 100 mAU) | | |
|---|---|---|---|---|
| | | % Recovery by AU | % Recovery by Activity | % Recovery by ELISA |
| Phenyl (1.1 M NaCl) | Buffer: 96.0 Load: 89.8 | 3.3 | LOQ* | 0.4 |
| Phenyl (1.2 M NaCl) | Buffer: 104.2 Load: 97.9 | 2.3 | LOQ* | 0.1 |
| Phenyl (1.3 M NaCl) | Buffer: 110.3 Load: 103.7 | 2.1 | LOQ* | 0.0 |
| Phenyl (1.5 M NaCl) | Buffer: 123.1 Load: 116.5 | 1.7 | LOQ* | 0.0 |

| | | | | |
|---|---|---|---|---|
| • LOQ is equivalent to < 150 U/mL | | | | |

**TABLE 6 Results of the Phenyl Runs -Elution Evalaution**

| | | Eluate 100 mAU - 50 mAU | | | | | |
|---|---|---|---|---|---|---|---|
| | | Volume (CV) | % Recovery by AU | % Recovery by Activity | % Recovery by ELISA | Total HCP (ng) | HCP Fold Clearance |
| Phenyl (1.1 M NaCl) | | 5.8 | 83.0 | 85.6 | 80.4 | 215186 | 42 |
| Phenyl (1.2 M NaCl) | | 5.9 | 83.9 | 87.0 | 87.3 | 205548 | 45 |
| Phenyl (1.3 M NaCl) | | 5.9 | 83.9 | 88.2 | 87.8 | 271155 | 36 |
| Phenyl (1.5 M NaCl) | | 6.0 | 85.0 | 90.4 | 89.7 | 242555 | 40 |
| | | | | | | | |

| | Buffer Conductivit y (mS/cm) | Eluate (100 mAU - 50 mAU) | | | | | |
|---|---|---|---|---|---|---|---|
| | | Volume (CV) | % Recovery by AU | % Recovery by Activity | % Recovery by ELISA | Total HCP (ng) | HCP Fold Clearance |
| Phenyl (180 mM NaCl) | 119.5 | 5.7 | 82.9 | 87.2 | 88.1 | 202367 | 45 |
| Phenyl (200 mM NaCl) | 21.0 | 5.9 | 83.9 | 87.0 | 81.3 | 205548 | 45 |
| Phenyl (220 mM NaCl) | 22.9 | 6.0 | 83.4 | 85.3 | 86.7 | 220549 | 42 |

Loading at 1.1 M, 1.2 M, 1.3 M, and 1.5 M NaCl, pH 7.0 (conductivity range from 90 to 117 mS/cm at 25'C), resulted in similar HNS loss in FT/Wash, elution volumes, recoveries, and HCP clearance of the Phenyl eluate (Table 5). Elution of the Phenyl column, at 180 mM, 200 mM, and 220 mM NaCl, pH 7.0 (conductivity range from 20 to 23 mS/cm at 25°C), resulted in similar elution volumes, recoveries, and HCP clearance of the Phenyl eluate (Table 6) .

Based on these results, adjusting the Phenyl load to 1.2 M NaCl, pH 7.0 + 1.0 with a conductivity range of 90 to 110 mS/cm at 25°C may be preferred. A recommended Phenyl equilibration and wash buffer is 20 mM MES-Tris, 1.2 M NaCl, pH 7.0 + 1.0 with a conductivity range of 100 to 120 mS/cm at 25°C. A recommended Phenyl elution buffer is 20 mM MES-Tris, 200 mM NaCl, pH 7.0 + 1.0 with a conductivity range of 19 to 23 mS/cm at 25°C.

### Example 3

### Ceramic Hydroxyapatite (HA) Column

HA loading and elution of a different sodium phosphate concentrations and different pH values were studied. The operational conditions for the experimental runs are summarized in Tables 7 and 8. The flow rate for the HA runs was at 150 cm/hr.

**TABLE 7 Operation Conditions of the HA Runs - Load Evaluation**

| Process Steps | HA Load at 2 mM NaPO₄, pH 7.0 (Control) | HA Load at 2 mM NaPO₄, pH 7.0 (Control) | HA Load at 2 mM NaPO₄, pH 7.0 (Control) |
|---|---|---|---|
| Column Size | 1.0 X 24.5 cm | | |
| Starting Material | Phenyl Eluate Pool (Phenyl 1-Phenyl 6) | | |
| Sanitization | 0.5 M NaOH | | |
| Equilibration | 2 mM NaPO₄ 20 mM MES-Tris, 200 mM NaCl, pH 7.0 | 4 mM NaPO₄ 20 mM MES-Tris, 200 mM NaCl, pH 7.0 | 2 mM NaPO₄ 20 mM MES-Tris, 200 mM NaCl, pH 7.2 |
| Load | • Adjusted Phenyl pool to 2 mM NaPO₄ | • Adjusted Phenyl pool to 4 mM NaPO₄ | • Adjusted Phenyl pool to 2 mM NaPO₄ and |
| | • 0.2 µm filtered | • 0.2 µm filtered | pH 7.2 • 0.2 µm |
| | • Loaded at 7.6 AU/mL resin | • Loaded at 7.6 AU/mL resin | filtered • Loaded at 7.6 AU/mL resin |
| Wash | 4 CV of 2 mM NaPO₄ 20 mM MES-Tris, 200 mM NaCl, pH 7.0 | 4 CV of 4 mM NaPO₄ 20 mM MES-Tris, 200 mM NaCl, pH 7.0 | 4 CV of 2 mM NaPO₄ 20 mM MES-Tris, 200 mM NaCl, pH 7.2 |
| Elute | • 25 mM NaPO₄, pH 7.0 | | |
| | • Collect based on 100 mAU - 50 mAU setpoints | | |
| Strip | 2.5 CV of 250 mM NaPO₄, pH 7.0 | | |
| Clean | 2 CV of 0.5 M NaOH | | |

| | | | |
|---|---|---|---|
| Flowrate for the HA runs is at 150 cm/hour | | | |

**TABLE 8 Operation Conditions of the HA Runs - Elution evaluation**

| Process Steps | HA elution at 25 mM PO₄, pH 7.5 (Control) | HA elution at 20 mM PO₄, pH 7.5 | HA elution at 30 mM PO₄, pH 7.5 | HA elution at 25 mM PO₄, pH 7.4 | HA elution at 25 mM PO₄, pH 7.6 |
|---|---|---|---|---|---|
| Column Size | 1.0 cm X 24.5 cm | | | | |
| Starting Material | Phenyl Eluate Pool (Phenyl 1 - Phenyl6) | | | | |
| Sanitization | 0.5 M NaOH | | | | |
| Equilibration | 2 mM NaPO₄ 20 mM MES-Tris, 200 mM NaCl, pH 7.0 | | | | |
| Load | • Adjusted Phenyl Pool to 2 mM NaPO₄ | | | | |
| | • 0.2 µm filtered | | | | |
| | • Loaded at 7.67 AU/mL resin | | | | |
| Wash | 4 CV 2 mM NaPO₄ 20 mM MES-Tris, 200 mM NaCl, pH 7.0 | | | | |
| Elute | • 25 mM NAPO₄, pH 7.5 | • 20 mM NaPO₄, pH 7.5 | • 30 mM NaPO₄, pH 7.5 | • 25 mM NaPO₄, pH 7.4 | • 25 mM NaPO₄, pH 7.6 |
| | • Collect based on 100 mAU - 50 mAU setpoin ts | • Collect based on 100 mAU - 50 mAU setpoin ts | • Collect based on 100 mAU - 50 mAU setpoin ts | • Collect based on 100 mAU - 50 mAU setpoin ts | • Collect based on 100 mAU - 50 mAU setpoin ts |
| Strip | 2.5 CV of 250 mM NaPO₄, pH 7.0 | | | | |
| Clean | 2 CV of 0.5 M NaOH | | | | |

### Results

The results from the HA runs are summarized in Tables 9 and 10. The level of quantitation (LOC) is equivalent to < 150 U/ml.

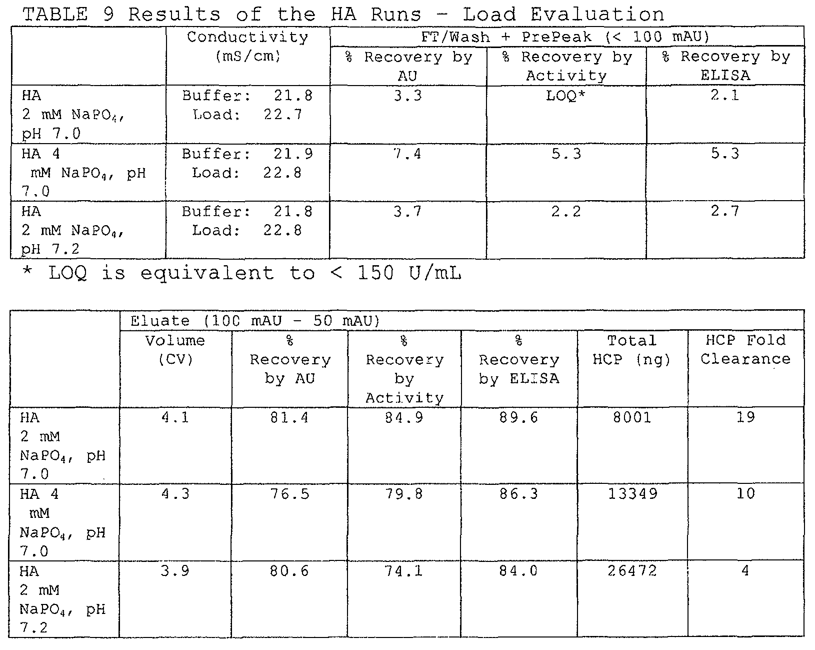

**TABLE 10 Results of the HA Runs -Elution Evaluation**

| | Buffer conductivity (mS/cm) | Eluate (100 mAU - 50 mAU) | | | | | |
|---|---|---|---|---|---|---|---|
| | | Volume (CV) | % Recovery by AU | % Recovery by Activity | 78.0 | Total HCP (ng) | HCP Fold Clearance |
| HA 20 mM NaPO₄, pH 7.5 | 3.18 | 4.6 | 75.9 | 78.1 | 88.1 | 7806 | 19 |
| HA 25 mM NaPO₄, pH 7.5 | 3.88 | 4.1 | 81.4 | 84.9 | 89.6 | 8001 | 19 |
| HA 30 mM NaPO₄, pH 7.5 | 4.59 | 4.0 | 81.8 | 83.8 | 91.5 | 17654 | 8 |
| HA 25 mM NaPO₄, pH 7.4 | 3.79 | 4.5 | 78.7 | 81.2 | 82.3 | 11834 | 13 |
| HA 25 mM NaPO₄, pH 7.6 | 3.96 | 4.5 | 80.6 | 84.2 | 89.8 | 18429 | 8 |

Loading the HA column at 4 mM NaPO₄ resulted in higher percent loss in absorbance units in the FT/Wash and lower percent recover by absorbance in the eluate (Table 9). At the HA state, the purity of the HA eluate is above 95% and recovery by absorbance units is more reliable than recovery based on activity or ELISA results, due to the relatively large variability in these assays. Loading the HA column at 2 mM NaPO₄, pH 7.2 resulted in lower HCP clearance in the eluate. Based on these results, the HA process appeared to be sensitive to phosphate concentration and pH. Adjusting the HA load to 2 mM NaPO₄, pH 7.0 ± 0.1 may be preferred. HA equilibration and wash buffer of 2 mM NaPO₄, 20 mM MES-Tris, 200 mM NaCl, pH 7.0 ± 0.1 also may be preferred.

Eluting the HA column with a 20 mM NaPO₄, pH 7.5 buffer resulted in lower percent recovery as measured by absorbance units (Table 10). Increasing phosphate concentration to 30 mM increased the percent recovery but reduced the HCP clearance in the eluate. It was also noticed that changing the pH of the elution buffer will also reduce the HCP clearance in the eluates. A recommended HA elution buffer is 20 mM NaPO₄, pH 7.5 + 0.1.

### Example 4

### SP Sepharose Column

SP loads at 3.0 and 4.0 mS/cm and SP EQ/Wash at 20 mM NaCl and 40 mM NaCl were studied. SP elution at different salt concentrations (80 mM NaCl, 90 mM NaCl, 100mM NaCl) and different pH values (pH 4.9, pH 5.0, and pH 5.1) were also studied. The operational conditions for the experimental runs are summarized in Tables 11 and 12. The flow rate for the SP runs was 150 cm/hour.

**TABLE 11 Operation Conditions of the SP Runs - Load Evaluation**

| Process Steps | SP load at 4.0 mS/cm (undiluted) and EQ/Wash at 40 mM NaCl | SP load at 3.0 mS/cm (Control) and EQ/Wash at 20 mM NaCl |
|---|---|---|
| Column Size | 1.0 cm X 17.8 cm | |
| Starting Material | HA Eluate Pool (HA1 -HA7) | |
| Sanitization | 0.5 M NaOH | |
| Equilibration | 50 mM NaAcetate, 40 mM NaCl, pH 5.0 | 50 mM NaAcetate, 20 mM NaCl, pH 5.0 |
| Load | • Adjusted pH to 5.0 | • Adjusted pH to 5.0 and conductivity to 3.0 mS/cm |
| | • 0.2 µm filtered | |
| | • loaded with 8.5 mAU/mL resin | |
| | | • 0.2 µm filtered |
| | | • loaded with 8.5 mAU/mL resin |
| Wash | 4 CV of 50 mM NaAcetate, 40 mM NaCl, pH 5.0 | 4 CV of 50 mM NaAcetate, 20 mM NaCl, pH 5.0 |
| Elute | • 50 mM NaAcetate, 90 mM NaCl, pH 5.0 | |
| | • Collect based on 50 mAU - 50 mAU setpoints | |
| Strip | 2.5 CV of 50 mM NaAcetate, 1 M NaCl, pH 5.0 | |
| Clean | 2 CV of 0.5 M NaOH | |

| | | |
|---|---|---|
| The flowrate for SP runs is at 150 cm/hour. | | |

**TABLE 12 Operation Conditions of the SP runs -Elution Evaluation**

| Process Steps | SP elution 90 mM NaCl, pH 5.0 (Control) | SP elution 80 mM NaCl, pH 5.0 | SP elution 100mM NaCl, pH 5.0 | SP elution 90 mM NaCl, pH 4.5 | SP elution 90 mM NaCl, pH 5.1 |
|---|---|---|---|---|---|
| Column Size | 1.0 cm X 17.8 cm | | | | |
| Starting Material | HA Eluate Pool (HA1 -HA7) | | | | |
| Sanitization | 0.5 M NaOH | | | | |
| Equilibration | 50 mM NaAcetate, 20 mM NaCl, pH 5.0 | | | | |
| Load | • Adjusted pH to 5.0 and conductivity to 3.0 mS/cm | | | | |
| | • 0.2 µm filtered | | | | |
| | • loaded with 8.5 mAU/mL resin | | | | |
| Wash | 4 CV of 50 mM NaAcetate, 20 mM NaCl, pH 5.0 | | | | |
| Elute | • 50 mM NaAcetat e, 90 mM NaCl, pH 5.0 | • 50 mM NaAcetat e, 80 mM NaCl, pH 5.0 | • 50 mM NaAcetat e, 100 mM NaCl, pH 5.0 | • 50 mM NaAcetat e, 90 mM NaCl, pH 4.9 | • 50 mM NaAcetat e, 40 mM NaCl, pH 5.1 |
| | • Collect based on 50 mAU - 50 mAU setpoint s | • Collect based on 50 mAU - 50 mAU setpoint s | • Collect based on 50 mAU - 50 mAU setpoint s | • Collect based on 50 mAU - 50 mAU setpoint s | • Collect based on 50 mAU - 50 mAU setpoint s |
| Strip | 2.5 CV of 50 mM NaAcetate, 1 M NaCl, pH 5.0 | | | | |
| Clean | 2 CV of 0.5 M NaOH | | | | |

| | | | | | |
|---|---|---|---|---|---|
| The flowrate for SP runs is at 150 cm/hour. | | | | | |

The results from the SP runs are summarized in Tables 13 and 14.

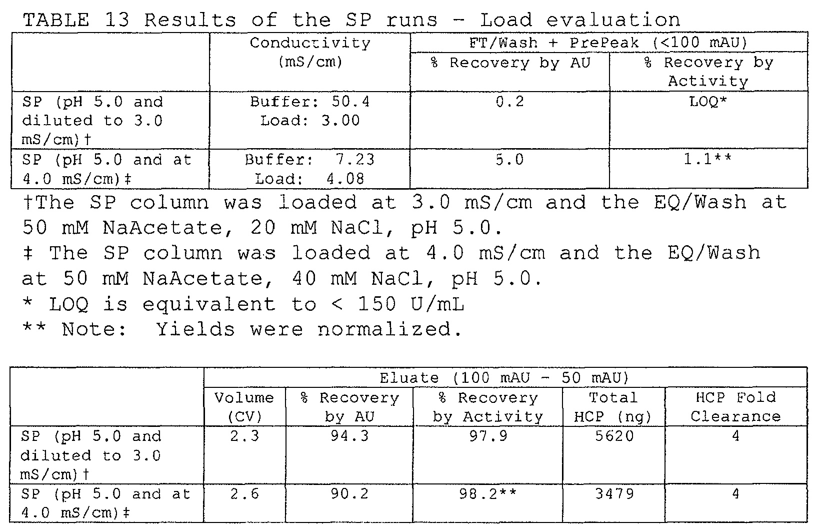

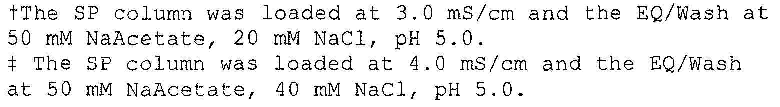

**TABLE 14 Results of the SP Runs -Elution Evaluation**

| | Buffer conductivity (mS/cm) | Eluate (100 mAU - 50 mAU) | | | | |
|---|---|---|---|---|---|---|
| | | Volume (CV) | % Recovery by AU | % Recovery by Activity | Total HCP (ng) | HCP Fold Clearance |
| SP (80 mM NaCl, pH 5.0) | 11.5 | 4.7 | 90.0 | 90.8 | 4684 | 5 |
| SP (90 mM NaCl, pH 5.0) | 12.7 | 2.3 | 94.3 | 97.9 | 5620 | 4 |
| SP (100 mM NaCl, pH 5.0) | 13.7 | 1.4 | 95.6 | 95.3 | 4167 | 5 |
| SP (90 mM NaCl, pH 4.9) | 12.5 | 4.4 | 89.3 | 89.8 | 4088 | 5 |
| SP (90 mM NaCl, pH 5.1) | 12.8 | 1.3 | 95.3 | 97.7 | 4369 | 5 |

Loading the SP column with loads under diluted or undiluted conditions (conductivity range from 3.0 to 4.0 mS/cm) resulted in similar recoveries and HCP clearance of the SP eluate (Table 13). Washing the SP column at 20 mM or 40 mM NaCl, pH 5.0 (conductivity range from 5.0 to 7.2 mS/cm at 25°C) resulted in similar recoveries and HCP clearance of the SP eluate (Table 13). Based on these results, adjusting SP load to pH 5.0 + 0.1 with a conductivity range of 3.5 + 0.5 mS/cm for loading may be preferred. A recommended SP equilibration and wash buffer is 50 mM NaAcetate, 20 mM NaCl, pH 5.0 + 0.1 with a conductivity range of 5 to 7 mS/cm.

Eluting the SP column at 80 mM, 90 mM, and 100 mM NaCl, pH 5.0 (conductivity range from 11.5 to 13.7 mS/cm at 25°C) resulted in similar recoveries and HCP clearance (Table 14). However, the column volume of the eluate at 80 mM NaCl was 4.7 CV compared to 2.3 CV at 90 mM NaCl. Also, eluting the SP column at 90 mM NaCl, pH 4.9, pH 5.0, and pH 5.1 resulted in similar recoveries and HCP clearance. However, the eluate volume of the pH 4.9 elution was 4.4 CV compared to 2.3 CV of the eluate at pH 5.0. In both cases, the increase in volume of the eluate was due to tailing of the elution profile. To avoid collecting an excessive peak tail, it is recommended to collect the eluate from 50 mAU to 50 mAU or to a maximum of 3 CV, whichever comes first. A recommended SP elution buffer is 50 mM NaAcetate, 90 mM NaCl, pH 5.0 + 0.1 with a conductivity range of 12 to 14 mS/cm and peak collection of the eluate from 50 mAU to 50 mAU or to a maximum of 3 CV, whichever comes first.

These assays indicate that the protocols described above for preparing recombinant lysosomal sulfatase enzymes provide an efficient method for production of large quantities of highly purified enzyme, human Heparan-N-sulfatase (HNS).

## Claims

1. A process for the purification of human heparan-N-sulfatase (HNS) that contains reduced amounts of high pI HNS, comprising:
a) contacting a bulk HNS composition containing a mixture of human HNS and other contaminating proteins of a cell culture supernatant medium obtained from a host cell genetically engineered to make human HNS with an anionic exchange chromatography resin; and
b) extracting and/or purifying HNS from the bulk HNS composition from the anionic exchange chromatography resin by adjusting the pH to 7.0;
wherein the process removes from 10% to 25% of high pI HNS present in the bulk HNS composition.

2. The process of claim 1, wherein the removal of the high pI HNS leads to improved solubility of the extracted and/or purified HNS.

3. The process of any one of the preceding claims, wherein the process comprises washing the anionic exchange column with 5 column volumes of a buffer containing 20 mM MES-Tris and 20 mM NaCl at a pH of 7.0 prior to extracting HNS.

4. The process of any one of the preceding claims, wherein the process comprises eluting HNS from the anionic exchange chromatography resin using a buffer constituting 20mM MES-Tris and 180mM NaCl at pH 7.0.

5. The process of any one of the preceding claims, wherein conductivity of the bulk HNS composition is adjusted to obtain a conductivity of from 3 to 4 mS/cm prior to contacting the bulk HNS composition with the anionic exchange chromatography resin.

6. The process of any one of the preceding claims, wherein the bulk HNS composition is subjected to viral inactivation prior to loading on the anionic exchange column.

7. The process of any one of the preceding claims, wherein the process further comprises contacting the extracted and/or purified HNS with a hydrophobic interaction chromatography resin, a hydroxyapatite chromatography resin, and/or a cationic exchange chromatography resin.

8. The process of claim 7, wherein the extracted and/or purified HNS obtained from the anionic exchange chromatography step is the starting material for a hydrophobic interaction step, optionally wherein the NaCl concentration of the extracted/purified HNS is adjusted to 1.1 M to 1.5 M NaCl prior to contacting the HNS composition with a hydrophobic interaction column.

9. The process of claim 8, wherein the HNS composition obtained from the hydrophobic interaction column is used as the starting material for purification employing a hydroxyapatite column, optionally wherein the NaPO₄ concentration of HNS composition after elution from the hydrophobic interaction column is adjusted to 2 mM to 4 mM.

10. The process of claim 9, wherein the HNS composition obtained from the hydroxyapatite column is used as the starting material for purification employing a cationic exchange column, optionally wherein conductivity of the HNS composition is adjusted to 3 to 4 mS/cm prior to loading on the cationic exchange column.

11. A human HNS composition obtained by the process of any one of claims 1-10.

12. A human HNS composition that contains reduced amounts of high pI HNS having an HNS concentration above 15 gram per liter, wherein the composition is produced by a process that removes from 10% to 25% of high pI HNS present in bulk HNS compositions.

## Patentansprüche

1. Verfahren zur Aufreinigung menschlicher Heparan-N-Sulfatase (HNS), die reduzierte Mengen an HNS mit hohem pI enthält, umfassend:
a) Inkontaktbringen einer HNS-Rohzusammensetzung, die ein Gemisch von menschlicher HNS und anderen, verunreinigenden Proteinen eines von einer zur Erzeugung von menschlicher HNS gentechnisch manipulierten Wirtszelle gewonnenen Zellkulturüberstandmediums enthält, mit einem Anionenaustauschchromatographieharz; und
b) Extrahieren und/oder Aufreinigen von HNS aus der HNS-Rohzusammensetzung vom Anionenaustauschchromatographieharz durch Einstellen des pH auf 7,0;
wobei durch das Verfahren 10% bis 25% von in der HNS-Rohzusammensetzung vorliegender HNS mit hohem pI entfernt werden.

2. Verfahren nach Anspruch 1, wobei die Abtrennung der HNS mit hohem pI zu einer verbesserten Löslichkeit der extrahierten und/oder aufgereinigten HNS führt.

3. Verfahren nach einem der vorhergehenden Ansprüche, wobei das Verfahren Waschen der Anionenaustauschsäule mit 5 Säulenvolumen eines Puffers, der 20 mM MES-Tris und 20 mM NaCl bei einem pH-Wert von 7,0 enthält, vor dem Extrahieren von HNS umfasst.

4. Verfahren nach einem der vorhergehenden Ansprüche, wobei das Verfahren Eluieren von HNS aus dem Anionenaustauschchromatographieharz unter Verwendung eines Puffers mit 20 mM MES-Tris und 180 mM NaCl bei pH 7,0 umfasst.

5. Verfahren nach einem der vorhergehenden Ansprüche, wobei die Leitfähigkeit der HNS-Rohzusammensetzung so eingestellt wird, dass eine Leitfähigkeit von 3 bis 4 mS/cm vor dem Inkontaktbringen der HNS-Rohzusammensetzung mit dem Anionenaustauschchromatographieharz erhalten wird.

6. Verfahren nach einem der vorhergehenden Ansprüche, wobei die HNS-Rohzusammensetzung einer Virusinaktivierung vor dem Auftragen auf die Anionenaustauschsäule unterzogen wird.

7. Verfahren nach einem der vorhergehenden Ansprüche, wobei das Verfahren ferner Inkontaktbringen der extrahierten und/oder aufgereinigten HNS mit einem Hydrophobe-Wechselwirkung-Chromatographieharz, einem Hydroxyapatitchromatographieharz und/oder einem Kationenaustauschchromatographieharz umfasst.

8. Verfahren nach Anspruch 7, wobei die aus dem Anionenaustauschchromatographie-Schritt erhaltene extrahierte und/oder aufgereinigte HNS das Ausgangsmaterial für einen Hydrophobe-Wechselwirkung-Schritt darstellt, gegebenenfalls wobei die NaCl-Konzentration der extrahierten/aufgereinigten HNS auf 1,1 M bis 1,5 M NaCl vor Inkontaktbringen der HNS-Zusammensetzung mit einer Hydrophobe-Wechselwirkung-Säule eingestellt wird.

9. Verfahren nach Anspruch 8, wobei die von der Hydrophobe-Wechselwirkung-Säule erhaltene HNS-Zusammensetzung als Ausgangsmaterial zur Aufreinigung unter Einsatz einer Hydroxyapatitsäule verwendet wird, gegebenenfalls wobei die NaPO₄-Konzentration von HNS-Zusammensetzung nach Elution von der Hydrophobe-Wechselwirkung-Säule auf 2 mM bis 4 mM eingestellt wird.

10. Verfahren nach Anspruch 9, wobei die von der Hydroxyapatitsäule erhaltene HNS-Zusammensetzung als Ausgangsmaterial zur Aufreinigung unter Einsatz einer Kationenaustauschsäule verwendet wird, gegebenenfalls wobei die Leitfähigkeit der HNS-Zusammensetzung vor dem Auftragen auf die Kationenaustauschsäule auf 3 bis 4 mS/cm eingestellt wird.

11. Menschliche-HNS-Zusammensetzung, erhalten mit dem Verfahren nach einem der Ansprüche 1-10.

12. Menschliche-HNS-Zusammensetzung, die reduzierte Mengen an HNS mit hohem pI enthält, mit einer HNS-Konzentration über 15 Gramm pro Liter, wobei die Zusammensetzung durch ein Verfahren erzeugt wird, bei dem 10% bis 25% von in HNS-Rohzusammensetzungen vorliegender HNS mit hohem pI entfernt werden.

## Revendications

1. Procédé de purification d'héparane-N-sulfatase (HNS) humaine qui contient des quantités réduites de HNS à pI élevé, comprenant :
a) la mise en contact d'une composition de HNS brute contenant un mélange de HNS humaine et d'autres protéines contaminantes d'un milieu surnageant de culture de cellules obtenu à partir d'une cellule hôte génétiquement modifiée pour fabriquer HNS humaine avec une résine de chromatographie d'échange d'anions ; et
b) l'extraction et/ou la purification de HNS à partir de la composition de HNS brute à partir de la résine de chromatographie d'échange d'anions par ajustement du pH à 7,0 ;
le procédé éliminant de 10 % à 25 % de HNS à pI élevé présente dans la composition de HNS brute.

2. Procédé de la revendication 1, dans lequel l'élimination de la HNS à pI élevé conduit à une solubilité améliorée de la HNS extraite et/ou purifiée.

3. Procédé de l'une quelconque des revendications précédentes, le procédé comprenant le lavage de la colonne d'échange d'anions avec 5 volumes de colonne d'un tampon contenant MES-Tris 20 mM et NaCl 20 mM à un pH de 7,0 avant l'extraction de HNS.

4. Procédé de l'une quelconque des revendications précédentes, le procédé comprenant l'élution de HNS à partir de la résine de chromatographie d'échange d'anions au moyen d'un tampon constitué de MES-Tris 20 mM et NaCl 180 mM à pH 7,0.

5. Procédé de l'une quelconque des revendications précédentes, dans lequel la conductivité de la composition de HNS brute est ajustée pour obtenir une conductivité de 3 à 4 mS/cm avant la mise en contact de la composition de HNS brute avec la résine de chromatographie d'échange d'anions.

6. Procédé de l'une quelconque des revendications précédentes, dans lequel la composition de HNS brute est soumise à une inactivation virale avant chargement sur la colonne d'échange d'anions.

7. Procédé de l'une quelconque des revendications précédentes, le procédé comprenant en outre la mise en contact de la HNS extraite et/ou purifiée avec une résine de chromatographie d'interaction hydrophobe, une résine de chromatographie d'hydroxyapatite et/ou une résine de chromatographie d'échange de cations.

8. Procédé de la revendication 7, dans lequel la HNS extraite et/ou purifiée obtenue dans l'étape de chromatographie d'échange d'anions est le matériau de départ pour une étape d'interaction hydrophobe, facultativement dans laquelle la concentration de NaCl de la HNS extraite/purifiée est ajustée à 1,1 M à 1,5 M de NaCl avant mise en contact de la composition de HNS avec une colonne d'interaction hydrophobe.

9. Procédé de la revendication 8, dans lequel la composition HNS obtenue à partir de la colonne d'interaction hydrophobe est utilisée en tant que matériau de départ pour la purification en utilisant une colonne d'hydroxyapatite, facultativement dans lequel la concentration de NaPO₄ de la composition de HNS après élution depuis la colonne d'interaction hydrophobe est ajustée à 2 mM à 4 mM.

10. Procédé de la revendication 9, dans lequel la composition de HNS obtenue à partir de la colonne d'hydroxyapatite est utilisée en tant que matériau de départ pour la purification en utilisant une colonne d'échange de cations, facultativement dans lequel la conductivité de la composition de HNS est ajustée à 3 à 4 mS/cm avant chargement sur la colonne d'échange de cations.

11. Composition de HNS humaine obtenue par le procédé de l'une quelconque des revendications 1 à 10.

12. Composition de HNS humaine qui contient des quantités réduites de HNS à pI élevé ayant une concentration de HNS supérieure à 15 grammes par litre, la composition étant produite par un procédé qui élimine de 10 % à 25 % de HNS à pI élevé présente dans les compositions de HNS brutes.
